# EUROPEAN PATENT SPECIFICATION

(11) **EP 2 335 623 B1**
(45) Date of publication and mention of the grant of the patent: **04.07.2012**
(21) Application number: 10252141.6
(22) Date of filing: 17.12.2010
(51) Int. Cl.: A61B 17/34

(54) **Surgical access apparatus with constraining mechanism**
Chirurgische Zugriffsvorrichtung mit Sicherungsmechanismus
Appareil d'accès chirurgical avec mécanisme de contrainte

(30) Priority: 18.12.2009 US 287837 P; 07.12.2010 US 961526
(43) Date of publication of application: 22.06.2011
(73) Proprietor: Tyco Healthcare Group LP, New Haven, CT 06511 (US)
(72) Inventor: Brockmeier, Oivind, Medford, MA 02155 (US); Judson, Jared Alden, Medford, MA 02155 (US); Focht, Kenneth Allen, Needham, MA 02492 (US)
(74) Representative: Soames, Candida Jane

(56) References cited:
- EP-A1- 1 994 895
- US-A- 5 634 908

## Description

### BACKGROUND

### 1. Technical Field

The present disclosure relates to a surgical portal for accessing underlying body tissue to permit the introduction of surgical objects in conjunction with a medical procedure. More particularly, the present disclosure relates to a surgical portal including a constraining mechanism for effecting the positioning and/or stabilizing of a surgical object with respect to the surgical portal.

### 2. Discussion of Related Art

Surgical portals are employed in various minimally invasive procedures including laparoscopic or endoscopic procedures. Such portals are inclusive of trocar cannulas, catheters. These portals typically incorporate a seal to form a fluid tight seal about an instrument passed through the portal. These seals are often limited by their ability to sustain a seal when an instrument, particularly, a smaller diameter instrument, is moved off-axis relative to a central axis of the portal.

During a typical surgical procedure, several portals may be positioned to access an underlying body cavity. The clinician may move from portal to portal to perform the desired surgical task(s). One disadvantage of this approach is that an instrument within a portal may be unattended and subject to dislodgement, particularly, when accessing a pressurized body cavity.

The two part form of appended claim 1 is based on the disclosure of EP 1994895.

### SUMMARY

Accordingly, a surgical access apparatus having the ability to constrain movement of a surgical instrument to minimize dislodgement and potentially improve the sealing capacity is provided as in appended claim 1. In accordance with one embodiment, the surgical access apparatus includes a housing member, a portal member extending from the housing member and defining a longitudinal passage therethrough dimensioned to permit passage of a surgical object and a constraining mechanism associated with the portal member. The constraining mechanism includes a constraining member dimensioned and positioned to intersect the longitudinal passage. The constraining member is adapted to engage the surgical object and potentially retain the surgical object in fixed relation with respect to the central longitudinal axis. The constraining member may be adapted to bias the surgical object toward a position radially displaced relative to the central longitudinal axis. The constraining member is adapted to transition from an initial condition in the absence of the surgical object to a stressed condition in the presence of the surgical object, and may be normally biased toward the initial condition.

In one embodiment, the constraining member includes an elongated spring member. The elongated spring member may be at least partially disposed within the portal member. The elongated spring member includes leading and trailing ends. One of the leading and trailing ends may be mounted in fixed relation to the portal member. The other of the leading and trailing end may be mounted for longitudinal movement relative to the portal member. The elongated spring member may define a bowed segment between the leading and trailing ends, and may be dimensioned to engage and substantially restrain the surgical object against an inner wall surface of the portal member.

In another embodiment, the constraining member defines a substantially disc shaped member. The disc shaped member has an opening therethrough for substantially restraining the surgical object. The opening may be radially displaced with respect to the central longitudinal axis of the portal member. The disc shaped member further has inner surfaces defining an object passage in communication with the opening. The inner surfaces may be dimensioned and configured to bias the surgical object toward the opening. The inner surfaces may be arranged to taper inwardly toward the opening.

In another embodiment, the constraining member includes a lever mounted within one of the housing and the portal member. The lever may be mounted for pivotal movement between a first position corresponding to the initial condition of the constraining member and a second position corresponding to the stressed condition of the constraining member. The lever may be normally biased toward the first position. An eyelet may be associated with the constraining member. The eyelet may define an opening for passage of the surgical object. The lever may be dimensioned to engage and substantially restrain the surgical object against an inner wall surface of the eyelet. The lever may be dimensioned to engage the eyelet, and cooperate with the eyelet to substantially close the longitudinal passage of the portal member when in the first position thereof.

An object seal is disposed within the housing. The object seal is dimensioned to establish a substantial seal about the surgical object. A zero closure valve adapted to open to permit passage of the surgical object and to close in the absence of the surgical objet is disposed within the housing.

### BRIEF DESCRIPTION OF THE DRAWINGS

Preferred embodiments of the present disclosure will be better appreciated by reference to the drawings wherein:
**FIG. 1** is a perspective view of the surgical access apparatus in accordance with the principles of the present disclosure;
**FIG. 1A** is a side cross-sectional view of the surgical access apparatus taken along the lines 1A-1A of **FIG. 1****.**
**FIG. 2** is a side cross-sectional view of the surgical access apparatus taken along the lines **2-2** of **FIG. 1** illustrating the constraining mechanism in the form of an elongated spring and depicted in an initial condition in the absence of a surgical object;
**FIG. 3** is a top plan view of the elongated spring illustrating the mounting mechanism for mounting the elongated spring to the internal wall of the portal member;
**FIG. 4** is a perspective view of the elongated spring;
**FIG. 5** is a view similar to the view of **FIG. 4** illustrating the elongated spring in a stressed condition in the presence of a surgical object.
**FIG. 6** similar to the view of **FIG. 3** illustrating the arrangement of the mounting mechanism when the elongated spring is in the stressed condition;
**FIG. 7** is an axial view taken along the lines 7-7 of **FIG. 5** illustrating the elongated spring constraining the surgical object;
**FIG. 8** is an axial view illustrating the elongated spring constraining an instrument having a relatively small diameter;
**FIG. 9** is a cross-sectional view of an alternative embodiment of the present disclosure illustrating the constraining mechanism in the form of a constraining disc;
**FIG. 10** is a view similar to the view of **FIG. 9** illustrating introduction of a surgical object within the constraining disc of **FIG. 9****;**
**FIG. 11** is a view similar to the view of **FIG. 10** illustrating the constraining disc biasing the surgical object in a radial outward direction to be constrained within the radial displaced opening;
**FIG. 12** is a side cross sectional view of a segment of the portal member illustrating another alternate embodiment of the constraining mechanism in the form of a lever and associated constraining eyelet with the lever in a first position;
**FIG. 13** is a cross-sectional view taken along the lines **13-13** of **FIG. 12** illustrating the lever mounted within the constraining eyelet;
**FIG. 14** is a perspective view of the eyelet of the constraining mechanism of **FIGS. 12** and **13**;
**FIG. 15** is a partial cross-sectional view illustrating a surgical object passing through the constraining mechanism with the lever in a second pivoted position and biasing the surgical object radially outwardly against the constraining eyelet; and
**FIG. 16** is a perspective view further illustrating the surgical object secured the within the constraining mechanism.

### DETAILED DESCRIPTION OF THE EMBODIMENTS

The access apparatus of the present disclosure is capable of accommodating objects of varying diameters, e.g., including instruments from about 2.0 millimeter (mm) to about 15 millimeter (mm), during a minimally invasive surgical procedure. Moreover, the access apparatus 100 contemplates the introduction and manipulation of various types of instrumentation adapted for insertion through a trocar and/or cannula assembly while maintaining a fluid tight interface about the instrumentation to prevent gas and/or fluid leakage from the established pneumoperitoneum so as to preserve the atmospheric integrity of a surgical procedure. Specifically, the access apparatus 10 includes a constraining mechanism for selectively retraining the surgical object in a predetermined fixed position with respect to the apparatus. This feature of the present disclosure desirably minimizes the potential of inadvertent movement or dislodgement of the surgical object, and may assist in maintaining a seal within the apparatus.

Examples of surgical objects or instrumentation contemplated for use with the access apparatus 100 include clip appliers, graspers, dissectors, retractors, staplers, laser probes, photographic devices, endoscopes and laparoscopes, tubes, and the like. Such instruments will be collectively referred to herein as "surgical objects".

In the following discussion, the term "proximal" or "trailing" will refer to the portion of the access apparatus nearest to the clinician during the surgical procedure while the term "distal" or "leading" will refer to that portion of the access apparatus most remote to the clinician.

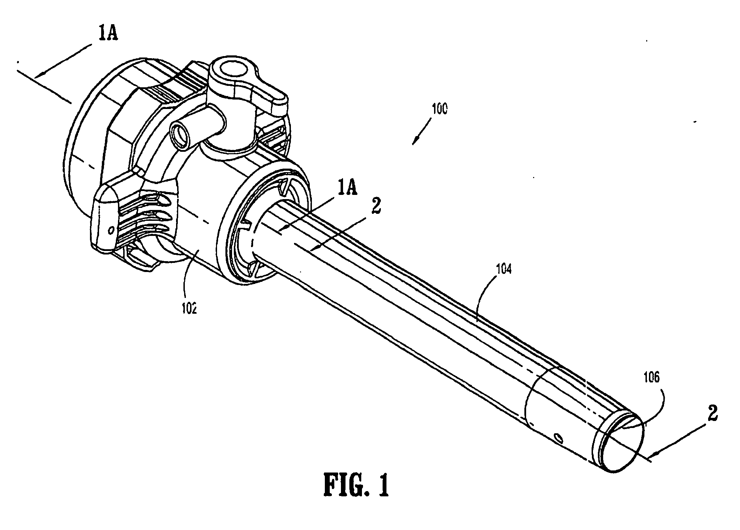

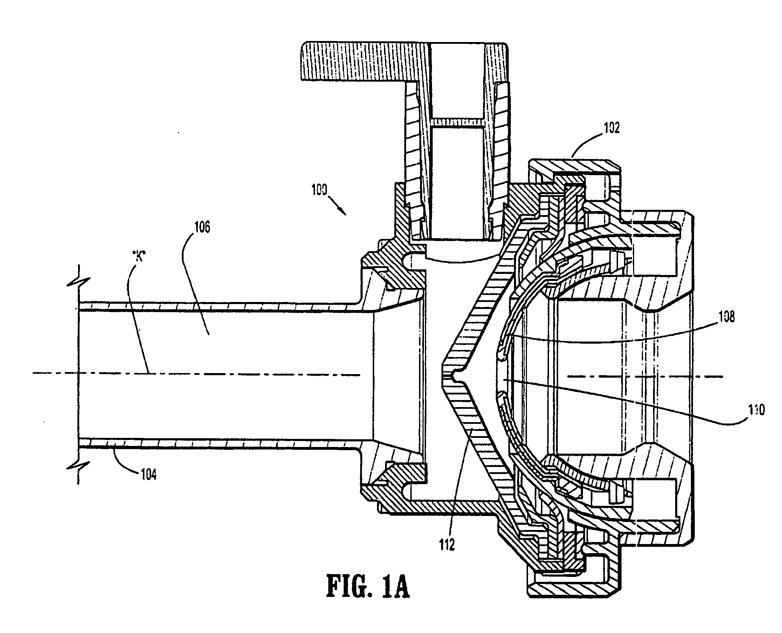

Referring now to the drawings, in which like reference numerals identify identical or substantially similar parts throughout the several views, **FIGS. 1** and **1A** illustrate the access apparatus **100** of the present disclosure. Access apparatus **100** may be any member suitable for the intended purpose of accessing a body cavity and typically defines a passageway permitting introduction of instruments or the clinician's hand therethrough. Access apparatus **100** is particularly adapted for use in laparoscopic surgery where the peritoneal cavity is insufflated with a suitable gas, e.g., CO₂, to raise the cavity wall from the internal organs therein. Access apparatus **100** is typically used with an obturator assembly (not shown) which may be blunt, a non-bladed, or a sharp pointed instrument positionable within the passageway of the access apparatus **100.** The obturator assembly is utilized to penetrate the abdominal wall to introduce the access apparatus **100** through the abdominal wall, and then subsequently is removed from the access apparatus **100** to permit introduction of the surgical instrumentation utilized to perform the procedure through the passageway.

Access apparatus **100** includes housing member **102** and portal member **104** connected to the housing member **102** and extending therefrom. Portal member **104** defines a longitudinal axis **"k"** extending along the length of the portal member **104.** Housing member **102** and portal member **104** further define internal longitudinal passage **106** dimensioned to permit passage of surgical instrumentation. Portal member **104** may be formed of any suitable medical grade material, such as stainless steel or other rigid materials, including polymeric materials, such as polycarbonate, or the like. Portal member **104** may be transparent or opaque. The diameter of portal member **104** may vary, but typically ranges from about **4.5** millimeters (mm) to about **15** millimeters (mm).

Housing member **102** may include a number of components assembled together to define the outer housing shown in the drawings. Housing member **102** includes object seal **108** which is adapted to establish a substantial seal about a surgical object, e.g., a surgical instrument. Object seal **108** may be a seal having an inner area or inner surfaces defining central aperture **110** for sealed reception of a surgical instrument. In one embodiment, object seal **108** is the gimbal seal disclosed in commonly assigned U.S. 2006 224120. Object seal **108** may comprise an elastomeric material and may, or may not, include a fabric layer juxtaposed with the elastomeric material. For example, in one embodiment, object seal **108** desirably comprises an elastomeric material compression-molded with a fabric material such as disclosed in certain embodiments of the commonly assigned U.S. Pat. No. 6,702,787 to Racenet. The fabric may comprise a woven, knitted, braided, or non-woven material of polymeric materials. Alternatively, object seal **108** may comprise a gel material fabricated from soft urethane gel, silicon gel, etc.

Housing member **102** further includes valve **112.** Valve **112** may be a zero-closure valve such as duck-bill valve having a slit which is adapted to close in the absence of a surgical object and/or in response to insufflation gases of the pressurized cavity. In the alternative, valve **112** may be a gel seal, balloon valve, or a flapper valve.

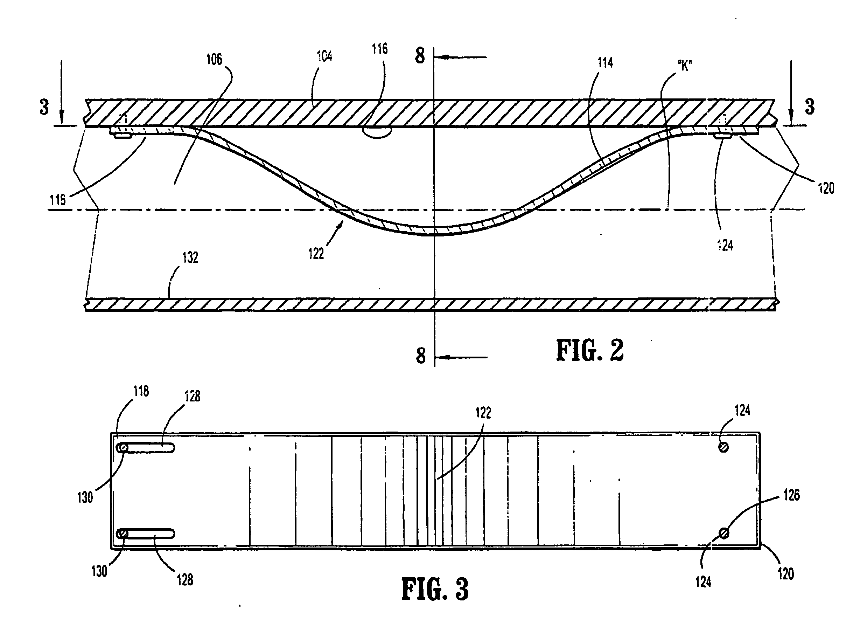

Referring now to **FIGS. 2-4****,** one embodiment of the constraining mechanism of the present disclosure is illustrated. Constraining mechanism is adapted to constrain or restrict movement of the surgical object "o" within portal member **104.** In embodiments, the constraining mechanism is adapted to bias the surgical object "o" in, e.g., a radial outward direction such that the surgical object "o" is restrained against structure within portal member **104.** In this manner, the surgical object "o" may be secured in a fixed longitudinal position within portal member **104** thereby minimizing the potential for the surgical object "o" to be dislodged or expelled from access apparatus **100.** In addition, the constraining mechanism may limit off-axis or angulated movement of the surgical object "o" thereby assisting in maintaining a seal with object seal **108** about the surgical object "o". Even further, the constraining mechanism will also permit the clinician to leave the surgical object unattended during the performance of other surgical tasks. In the embodiments of **FIGS. 2-4****,** the constraining mechanism includes elongated spring **114** mounted to an internal wall surface **116** of portal member **104.** Elongated spring **114** may comprise any suitable resilient material such as stainless steel, titanium or a resilient polymeric material. Elongated spring **114** includes leading (distal) and trailing (proximal) ends **118, 120** respectively and an intermediate segment **122** disposed between the leading and the trailing ends **118, 120.** Intermediate segment **122** includes a generally bowed segment having an arch or curvature extending toward the central longitudinal axis **"k".**

Elongated spring **114** may be mounted to portal member **104** through a variety of mechanisms. In one embodiment, trailing or proximal end **120** is secured to internal wall surface **116** with at least one fastener or pin **124,** which extends through a corresponding aperture **126** adjacent the trailing end. Two fasteners **124** and corresponding apertures **126** may be provided. Leading or distal end **118** is secured to internal wall surface **116** in a manner which permits the leading end **118** to move in a limited longitudinal direction with respect to longitudinal axis **"k".** For example, elongated spring **114** includes a pair of elongated slots **128** adjacent leading end **118.** Pins or fasteners **130** extend through slots **128** and are adapted to traverse the slots **128** during flexing of elongated spring **114.**

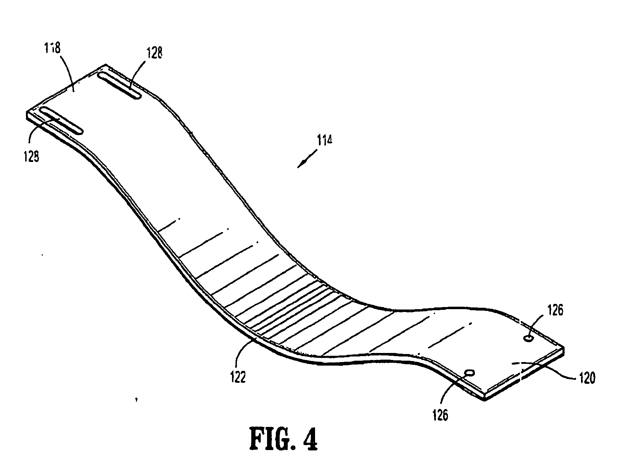

Elongated spring **114** is normally biased to an initial condition in which intermediate bowed segment **122** assumes the arrangement depicted in **FIGS. 2** and **4****.** Intermediate bowed segment **122** may depend outwardly such that it engages the opposed wall surface **132** of portal member **104.** In the alternative, bowed segment **122** may be displaced from opposed wall surface **132** when in the initial condition. Bowed segment **122** is dimensioned to intersect longitudinal passage **106** and the central longitudinal axis **"k"** to engage the surgical object.

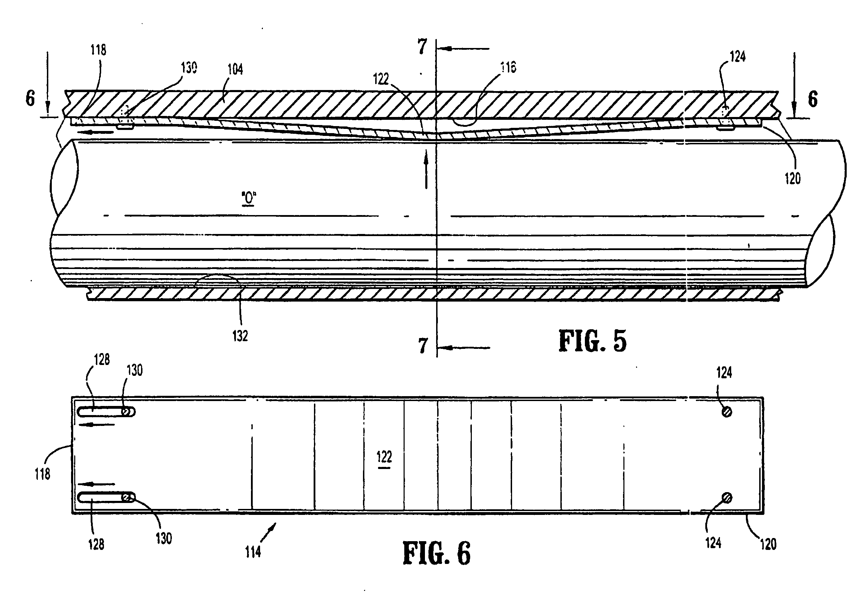

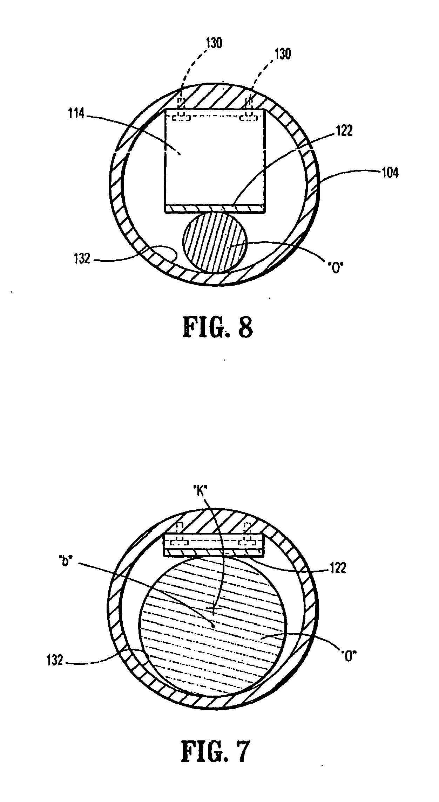

Referring now to **FIGS. 5-7****,** the operation of the constraining mechanism will be discussed. During the course of a surgical procedure, a surgical object "o" will be introduced through access apparatus **100.** As the surgical object "o" advances within portal member **104,** bowed segment **122** contacts or engages the surgical object "o". Further advancing movement of the surgical object "o" will cause bowed segment **122** to flex or deflect radially outwardly relative to central longitudinal axis "k" toward internal wall surface **116** and assume the stressed condition depicted in **FIGS. 5** and **7****.** Concurrently therewith, leading end **118** will move in a distal or leading direction to accommodate the deflection of bowed segment **122** with pins or fasteners **130** traversing elongated slots **128** of elongated spring **114.** As discussed, elongated spring **114** is normally biased to its initial condition. Accordingly, bowed segment **122** will continually urge the surgical object "o" in an opposed radial outward direction toward internal wall surface **132.** In one embodiment, bowed segment **122** traps or constrains the surgical object **"o"** against the opposed internal wall surface **132** of portal member **104** to secure the surgical object **"o"** at a predefined longitudinal position. In this position, the axis **"b"** of the surgical object **"o"** may be in general parallel relation with longitudinal axis **"k"** of portal member **104** which may assist in maintaining a seal about the surgical object **"o"** by resisting angulation of the surgical object **"o".** Upon removal of the surgical object **"o",** bowed segment **122** will assume its initial condition of **FIG. 2****.** **FIG. 8** illustrates bowed segment **122** acting in concert with internal wall surface **132** of portal member **104** to secure a surgical object **"o"** of a relatively small diameter.

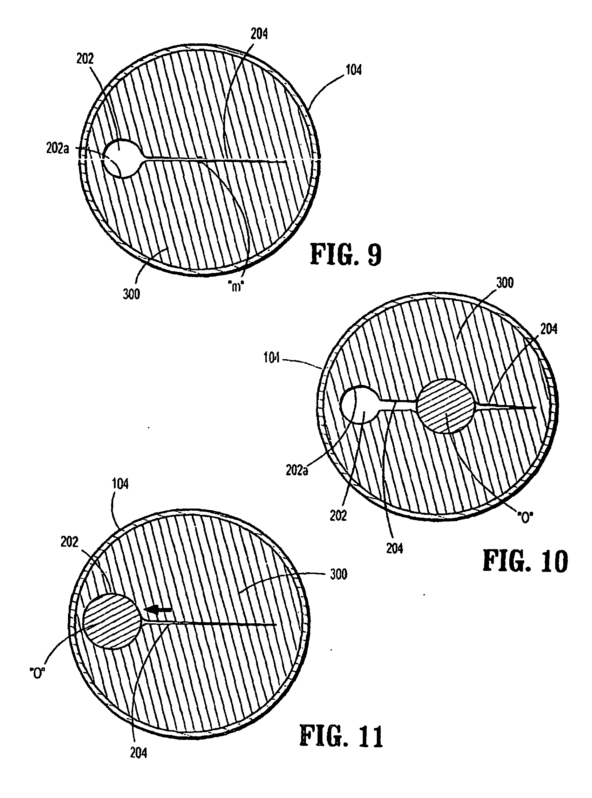

**FIGS. 9-11** illustrate another alternate embodiment of the present disclosure. In accordance with this embodiment, constraining member **200** is generally disk shaped and may be mounted to portal member **104** by any conventional means including adhesives, cements or mechanical means. Constraining member **200** may be secured to portal member **104** at a longitudinal fixed position. Constraining member **200** may be fabricated from any of the suitable resilient materials identified hereinabove in connection with the discussion of elongated spring. Constraining member **200** includes opening **202** and internal surfaces **204** communicating with the opening **202.** Opening **202** is radially displaced from the center **"m"** of constraining member **200** and with respect to the central longitudinal axis **"k"** of portal member **104.** Internal surfaces **204** may taper away from each other as the internal surfaces **204** approaches opening **202.** **FIG. 9** illustrates the initial condition of constraining member **200.** In this position, internal surfaces **204** are in an approximated relationship.

In use, surgical object **"o"** is introduced within the access apparatus **100** and advanced within portal member **104** to engage constraining member **200.** Internal surfaces **204** of constraining member **200** will deflect outwardly relative to accommodate passage of the surgical object **"o"** to assume the stressed condition depicted in **FIG. 10****.** However, because internal surfaces **204** are normally biased to the initial condition of **FIG. 9****,** the internal surfaces **204** act in concert to apply a radial force against the outer circumference of the surgical object **"o".** The application of the radial force about the circumference of the surgical object **"o"** will drive the surgical object "o" towards aperture **202,** and may be facilitated through the tapered arrangement (if present) of internal surfaces **204.** The surgical object **"o"** thereby is received within aperture **202** with the inner surfaces **202a** defining the aperture circumscribing the surgical object **"o".** The surgical object "o" is constrained within the aperture **202.** In one embodiment, the surgical object **"o"** may be secured at a longitudinal fixed position within aperture **202** through frictional engagement with inner surfaces **202a** defining aperture **202.** in the restrained condition, the axis **"b"** of the surgical object "o" may be in parallel relation with the longitudinal axis **"k"** of portal member **104,** thereby assisting in maintaining the seal about the surgical object **"o".**

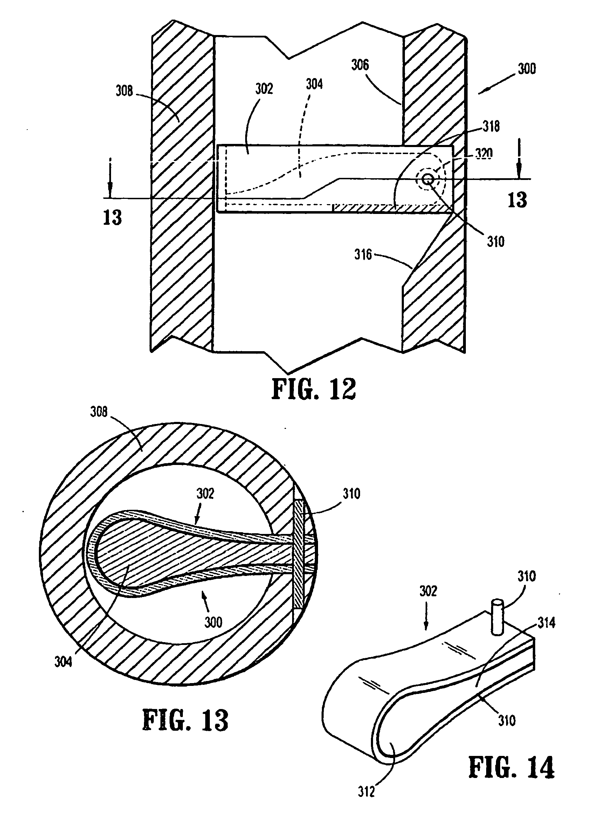

**FIGS. 12-14** illustrate another embodiment of the constraining mechanism **300** in accordance with the present disclosure. The constraining mechanism **300** includes eyelet **302** and lever **304** both of which are mounted within an internal surface **306** of portal member **308.** In one embodiment, both eyelet **302** and lever **304** define apertures for reception of mounting pin **308** which is secured to, or embedded within, portal member **308.** As best depicted in **FIG. 14****,** eyelet **302** defines an elongated or oblong opening **310** having a circular segment **312** displaced from mounting pin **308** and a tapered segment **314** adjacent the mounting pin 300.

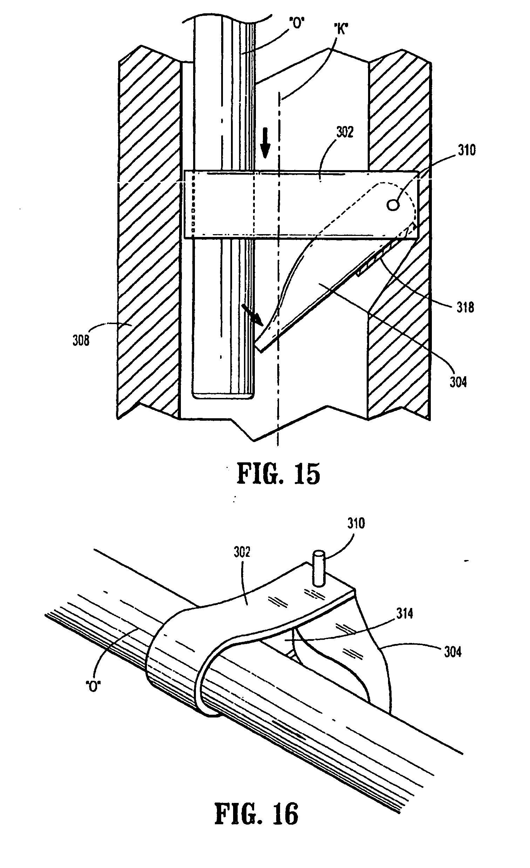

Lever **304** is adapted for pivotal movement about mounting pin **310** and relative to eyelet **302** between the initial condition depicted in **FIGS. 12-13** and the pivoted position depicted in **FIGS. 15** and **16****.** In one embodiment, portal member **308** includes recessed section **316** to permit unobstructed pivoting movement of lever **304.** Lever **304** is normally biased toward the initial condition through a biasing mechanism. For example, in one embodiment, a leaf spring **318** is mounted to portal member **308** and engages the underside of lever **304.** Leaf spring **318** will bias lever **304** in a vertical upward or counterclockwise direction **(****FIG. 12****)** i.e., toward the initial condition. In another embodiment, a torsion spring **320** (shown in phantom) may be coaxially arranged about mounting pin **310.** Torsion spring **320** may include one end mounted or embedded within portal member **104** and another end engageable with the underside of lever **304.** In a further embodiment, lever **304** is monolithically formed with portal member **104** and pivots about a living hinge between the initial and stressed conditions. Lever **304** may be fabricated from a suitable metal or polymeric material.

In use, the surgical object **"o"** is introduced through eyelet **302** and engages lever **304.** Lever **304** is deflected to pivot about mounting pin **308** to assume the stressed condition and permit passage of the surgical object **"o"** as depicted in **FIG. 15****.** The normal bias of lever **304** toward the initial condition will cause the lever **304** to engage and drive the surgical object **"o"** radially outwardly relative to the central axis **"k"** of portal member **104** and position the surgical object **"o"** within the circular segment **312** of eyelet **302.** Circular segment **302** may be dimensioned to at least partially circumscribe the surgical object **"o"** to further restrain the surgical object **"o"** within the circular segment **302** of eyelet **302.** Circular segment **302** may also be dimensioned to frictionally engage the surgical object **"o"** in a manner to secure, either individually, or in concert with lever **304,** the surgical object **"o"** at a fixed position within portal member **308** and relative to the longitudinal axis **"k",** and possibly parallel with the longitudinal axis **"k".** Once the surgical procedure or task is complete, the surgical object **"o"** may be withdrawn from portal member to permit lever **304** to assume the initial condition of **FIG. 12****.** In the initial condition, eyelet **302** and lever **304** may function as a closure valve preventing the escape of gases form the underlying body cavity

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the claims.

## Claims

1. A surgical access apparatus, which comprises:
a housing member (102);
an object seal (108) within the housing, the object seal dimensioned to establish a substantial seal about a surgical object suitable for carrying out a minimally invasive surgical procedure;
a zero closure valve (112) within the housing, adapted to open to permit passage of the surgical object and to close in the absence of the surgical object;
a portal member (104, 308) extending from the housing member and defining a central longitudinal axis, the housing member and the portal member defining a longitudinal passage (106) therethrough dimensioned to permit passage of the surgical object; and
a constraining mechanism including a constraining member (200, 300) adapted to engage the surgical object and bias the surgical object toward a position radially displaced relative to the central longitudinal axis; **characterized in that**
the constraining member is mounted to an internal wall surface (116) of the portal member (104) and dimensioned and positioned to intersect the longitudinal passage (106).

2. The surgical access apparatus according to claim **1** wherein the constraining member is adapted to transition from an initial condition in the absence of the surgical object to a stressed condition in the presence of the surgical object, the constraining member normally biased toward the initial condition.

3. The surgical access apparatus according to claim **2** wherein the constraining member includes an elongated spring member (114).

4. The surgical access apparatus according to claim 3 wherein the elongated spring member includes leading and trailing ends (118, 120), one of the leading and trailing ends mounted in fixed relation to the portal member, the other of the leading and trailing end mounted for longitudinal movement relative to the portal member.

5. The surgical access apparatus according to claim **4** wherein the elongated spring member defines a bowed segment (122) between the leading and trailing ends.

6. The surgical access apparatus according to claim **4 or 5** wherein the elongated spring member is dimensioned to engage and substantially restrain the surgical object against an inner wall surface of the portal member.

7. The surgical access apparatus according to claim 1 or claim 2 wherein the constraining member defines a substantially disc shaped member (200), the disc shaped member defining an opening (202) therethrough for substantially restraining the surgical object, the opening radially displaced with respect to the central longitudinal axis of the portal member, the disc shaped member further having inner surfaces (204) defining an object passage in communication with the opening, the inner surfaces dimensioned and configured to bias the surgical object toward the opening.

8. The surgical access apparatus according to claim 7 wherein the inner surfaces are arranged in to taper inwardly toward the opening.

9. The surgical access apparatus according to claim **1** or claim **2** wherein the constraining member (300) includes a lever (304), the lever mounted for pivotal movement between a first position corresponding to the initial condition of the constraining member and a second position corresponding to the stressed condition of the constraining member.

10. The surgical access apparatus according to claim 9 wherein the lever is normally biased toward the first position.

11. The surgical access apparatus according to claim **10** including an eyelet (302) defining an opening for passage of the surgical object, the lever dimensioned to engage and substantially restrain the surgical object against an inner wall surface of the eyelet.

12. The surgical access apparatus according to claim **11** wherein the lever is dimensioned to engage the eyelet, the lever and the eyelet being dimensioned to substantially close the longitudinal passage of the portal member.

## Patentansprüche

1. Chirurgisches Zugangsgerät, das Folgendes umfasst:
ein Gehäuseelement (102);
eine Gegenstandsversiegelung (108) im Gehäuse, wobei die Gegenstandsversiegelung abgemessen ist, um im Wesentlichen eine Versiegelung um einen verlängerten röhrenförmigen chirurgischen Gegenstand zu bilden;
ein Nullverschlussventil (112) im Gehäuse, das dazu ausgelegt ist, sich zu öffnen, um den Durchgang des chirurgischen Gegenstands zu ermöglichen, und sich in Abwesenheit des chirurgischen Gegenstands zu schließen;
ein röhrenförmiges Portalelement (104, 308), das sich vom Gehäuseelement erstreckt und eine zentrale Längsachse definiert, wobei das Gehäuseelement und das Portalelement einen Längsdurchgang (106) **dadurch** definieren, der abgemessen ist, um den Durchgang eines chirurgischen Gegenstandes zu ermöglichen; und
einen Sicherungsmechanismus, der ein Sicherungselement (200, 300) umfasst, dazu ausgelegt, in den chirurgischen Gegenstand einzugreifen und den chirurgischen Gegenstand in eine Position zu lenken, die mit Bezug auf die zentrale Längsachse radial verschoben ist; **dadurch gekennzeichnet, dass**
das Sicherungselement auf einer inneren Wandfläche (116) des Portalelements (104) montiert und abgemessen und angebracht ist, um den Längsdurchgang (106) zu schneiden.

2. Chirurgisches Zugangsgerät nach Anspruch 1, wobei das Sicherungselement ausgelegt ist, um sich von einem anfänglichen Zustand in Abwesenheit des chirurgischen Gegenstands in einen gespannten Zustand in Anwesenheit des chirurgischen Gegenstands zu bewegen, wobei das Sicherungselement normalerweise auf den anfänglichen Zustand gelenkt ist.

3. Chirurgisches Zugangsgerät nach Anspruch 2, wobei das Sicherungselement ein verlängertes Federelement (114) umfasst.

4. Chirurgisches Zugangsgerät nach Anspruch 3, wobei das verlängerte Federelement vordere und hintere Enden (118, 120) umfasst, wobei eines des vorderen und des hinteren Endes in einer festen Beziehung zum Portalelement montiert ist, wobei das andere des vorderen und des hinteren Endes für eine Längsbewegung mit Bezug auf das Portalelement montiert ist.

5. Chirurgisches Zugangsgerät nach Anspruch 4, wobei das verlängerte Federelement einen gekrümmten Abschnitt (122) zwischen dem vorderen und dem hinteren Ende definiert.

6. Chirurgisches Zugangsgerät nach Anspruch 4 oder 5, wobei das verlängerte Federelement abgemessen ist, um in den chirurgischen Gegenstand einzugreifen und diesen im Wesentlichen gegen eine innere Wandfläche des Portalelements zurückzuhalten.

7. Chirurgisches Zugangsgerät nach Anspruch 1 oder Anspruch 2, wobei das Sicherungselement ein im Wesentlichen scheibenförmiges Element (200) definiert, wobei das scheibenförmige Element eine Öffnung (202) **dadurch** definiert, um im Wesentlichen den chirurgischen Gegenstand zurückzuhalten, wobei die Öffnung mit Bezug auf die zentrale Längsachse des Portalelements radial versetzt ist, wobei das scheibenförmige Element weiter innere Flächen (204) aufweist, die einen Gegenstandsdurchgang definieren, der mit der Öffnung in Verbindung steht, wobei die inneren Flächen abgemessen und konfiguriert sind, um den chirurgischen Gegenstand auf die Öffnung zu lenken.

8. Chirurgisches Zugangsgerät nach Anspruch 7, wobei die inneren Flächen angeordnet sind, um sich nach Innen zur Öffnung hin zu verjüngen.

9. Chirurgisches Zugangsgerät nach Anspruch 1 oder Anspruch 2, wobei das Sicherungselement (300) einen Hebel (304) umfasst, wobei der Hebel zur Drehbewegung zwischen einer ersten Position, die dem anfänglichen Zustand des Sicherungselements entspricht, und einer zweiten Position, die dem gespannten Zustand des Sicherungselements entspricht, montiert ist.

10. Chirurgisches Zugangsgerät nach Anspruch 9, wobei der Hebel normalerweise auf die erste Position gelenkt ist.

11. Chirurgisches Zugangsgerät nach Anspruch 10, umfassend eine Öse (302), die eine Öffnung zum Durchgang des chirurgischen Gegenstands definiert, wobei der Hebel abgemessen ist, um in den chirurgischen Gegentand einzugreifen und diesen im Wesentlichen gegen eine innere Wandfläche der Öse zurückzuhalten.

12. Chirurgisches Zugangsgerät nach Anspruch 11, wobei der Hebel abgemessen ist, um in die Öse einzugreifen, wobei der Hebel und die Öse abgemessen sind, um im Wesentlichen den Längsdurchgang des Portalelements zu schließen.

## Revendications

1. Appareil d'accès chirurgical, comprenant :
un élément de logement (102) ;
un joint d'objet (108) dans le logement, le joint d'objet étant dimensionné de façon à établir un joint sensible autour de l'objet chirurgical allongé, tubulaire ;
une soupape de fermeture zéro (112) dans le logement, adaptée pour s'ouvrir de façon à permettre le passage de l'objet chirurgical et pour se fermer en l'absence dudit objet chirurgical ;
un élément de portique tubulaire (104, 308) s'étendant depuis l'élément de logement et définissant un axe longitudinal central, l'élément de logement et l'élément de portique définissant un passage longitudinal (106) à travers eux, dimensionné de façon à permettre le passage d'un objet chirurgical ; et
un mécanisme de contrainte, comprenant un élément de contrainte (200, 300) apte à mettre en prise l'objet chirurgical et à incliner ledit objet chirurgical vers une position radialement déplacée relativement à l'axe longitudinal central ; **caractérisé en ce que**
l'élément de contrainte est monté sur une surface de paroi interne (116) de l'élément de portique (104) et dimensionné et positionné de façon à couper le passage longitudinal (106).

2. Appareil d'accès chirurgical selon la revendication 1, dans lequel l'élément de contrainte est adapté pour passer d'une condition initiale en l'absence de l'objet chirurgical à une condition contrainte en présence de l'objet chirurgical, ledit élément de contrainte étant normalement incliné vers la condition initiale.

3. Appareil d'accès chirurgical selon la revendication 2, dans lequel l'élément de contrainte comprend un élément à ressort allongé (114).

4. Appareil d'accès chirurgical selon la revendication 3, dans lequel l'élément à ressort allongé comprend des extrémités avant et arrière (118,120), l'une des extrémités avant et arrière étant montée en relation fixe avec l'élément de portique, l'autre des extrémités avant et arrière étant montée de façon à permettre un mouvement longitudinal relativement à l'élément de portique.

5. Appareil d'accès chirurgical selon la revendication 4, dans lequel l'élément à ressort allongé définit un segment arqué (122) entre les extrémités avant et arrière.

6. Appareil d'accès chirurgical selon la revendication 4 ou 5, dans lequel l'élément à ressort allongé est dimensionné de façon à mettre en prise et à sensiblement contraindre l'objet chirurgical contre une surface de paroi interne de l'élément de portique.

7. Appareil d'accès chirurgical selon la revendication 1 ou 2, dans lequel l'élément de contrainte définit un élément sensiblement en forme de disque (200), l'élément en forme de disque définissant une ouverture (202) à travers lui pour contraindre sensiblement l'objet chirurgical, l'ouverture étant radialement déplacée relativement à l'axe longitudinal central de l'élément de portique, l'élément en forme de disque disposant en outre de surfaces internes (204) définissant un passage d'objet en communication avec l'ouverture, les surfaces internes étant dimensionnées et configurées pour incliner l'objet chirurgical vers l'ouverture.

8. Appareil d'accès chirurgical selon la revendication 7, dans lequel les surfaces internes sont disposées de façon à se rétrécir intérieurement vers l'ouverture.

9. Appareil d'accès chirurgical selon la revendication 1 ou 2, dans lequel l'élément de contrainte (300) comprend un levier (304), le levier étant monté, de façon à permettre un mouvement pivotant entre une première position correspondant à la condition initiale de l'élément de contrainte et une seconde position correspondant à la condition contrainte de l'élément de contrainte.

10. Appareil d'accès chirurgical selon la revendication 9, dans lequel le levier est normalement incliné vers la première position.

11. Appareil d'accès chirurgical selon la revendication 10, comprenant un oeillet (302) définissant une ouverture pour le passage de l'objet chirurgical, le levier étant dimensionné de façon à mettre en prise et à sensiblement contraindre l'objet chirurgical contre une surface de paroi interne de l'oeillet.

12. Appareil d'accès chirurgical selon la revendication 11 dans lequel le levier est dimensionné de façon à mettre en prise l'oeillet, le levier et l'oeillet étant dimensionnés de façon à fermer sensiblement le passage longitudinal de l'élément de portique.
